# EUROPEAN PATENT APPLICATION

(11) **EP 4 748 345 A1**
(43) Date of publication of application: **27.05.2026**
(21) Application number: 24843291.6
(22) Date of filing: 29.05.2024
(51) Int. Cl.: A61C 19/04, A61B 5/00, A61C 9/00, A61C 5/00, G06T 1/00, G06T 7/11, G06F 17/00, G06F 30/00

(54) **METHOD, APPARATUS, AND RECORDING MEDIUM FOR GENERATING INTRAORAL APPLIANCE**

(30) Priority: 19.07.2023 KR 20230093882; 07.05.2024 KR 20240059718
(71) Applicant: Medit Corp., Seoul 07207 (KR)
(72) Inventor: LEE, Jungkyen, Seoul 07207 (KR); LEE, Hotaik, Seoul 07207 (KR)
(74) Representative: MFG Patentanwälte Meyer-Wildhagen Meggle-Freund Gerhard PartG mbB
(86) International application number: PCT/KR2024/007295
(87) International publication number: WO 2025/018570

(57) **Abstract**

Disclosed is a technique for generating an intraoral appliance. A method performed by an electronic device according to one aspect of the present disclosure may include: identifying one or more target teeth based on at least one image of an oral cavity; generating first data on a first shape for an intraoral appliance for the one or more target teeth, based on the at least one image; generating second data on a second shape of the intraoral appliance, based on data on one or more antagonist teeth corresponding to the one or more target teeth and the first data; and generating third data on the intraoral appliance in which the first shape and the second shape are combined, based on the first data and the second data.

## Description

### TECHNICAL FIELD

The present disclosure relates to a technique for generating an intraoral appliance.

### BACKGROUND

A three-dimensional intraoral scanner is an optical device inserted into a patient's oral cavity and used to scan teeth, thereby obtaining a three-dimensional image of the oral cavity. By scanning the patient's oral cavity using a three-dimensional scanner, a plurality of two-dimensional images of the patient's oral cavity may be obtained, and the plurality of obtained two-dimensional images may be used to construct a three-dimensional image of the patient's oral cavity. For example, a dentist may insert a three-dimensional scanner into a patient's oral cavity and scan the patient's teeth, gingiva, and/or soft tissues, thereby obtaining a plurality of two-dimensional images of the patient's oral cavity. Thereafter, by applying three-dimensional modeling technology to the two-dimensional images of the patient's oral cavity, a three-dimensional image of the patient's oral cavity may be constructed.

The constructed three-dimensional image may be used to fabricate various intraoral appliances that are inserted into and mounted within the oral cavity to treat conditions such as temporomandibular joint disorders, teeth trauma, caries, and gum disease. For example, patients with a poorly aligned intraoral structure may easily experience temporomandibular joint fatigue and even develop temporomandibular joint disorders. Furthermore, an intraoral structure having uneven dentition in the teeth region or protruding gums may lead to bimaxillary prognathism. In this way, appliances may be designed to fix a patient's dentition in a specific shape in the short term, thereby preventing the patient from experiencing discomfort caused by the oral cavity structure. Alternatively, appliances may be designed to correct a patient's dentition to a specific shape in the long term. As another example, patients with oral habits, such as bruxism, may experience tooth damage. In this way, an appliance may be designed to protect the patient's teeth and to prevent the patient from experiencing discomfort or disorders caused by the patient's oral habits. As described above, intraoral appliances capable of being mounted in the patient's oral cavity for various purposes, such as tooth fixation, orthodontics, and tooth protection, may include splints. However, the technology for generating intraoral appliances applied to only some of the patient's teeth, such as a nociceptive trigeminal inhibition (NTI) splint or an anterior bite splint, has not been sufficiently developed.

### DISCLOSURE

### TECHNICAL PROBLEMS

A technical problem to be solved by at least one embodiment of the present disclosure is to provide a technology for automatically generating an intraoral appliance that is applied only to some of a patient's teeth.

A technical problem to be solved by at least one embodiment of the present disclosure is to provide a technology for determining a contour of an intraoral appliance on the basis of the shape of target teeth.

A technical problem to be solved by at least one embodiment of the present disclosure is to provide a technology for connecting an inner surface and an outer surface of an intraoral appliance.

A technical problem to be solved by at least one embodiment of the present disclosure is to provide a technology for determining a size of an additional part of an intraoral appliance.

A technical problem to be solved by at least one embodiment of the present disclosure is to provide a technology for determining a location of an additional portion of an intraoral appliance.

### TECHNICAL SOLUTION

A method performed by an electronic device according to one aspect of the present disclosure may include: identifying one or more target teeth based on at least one image of an oral cavity; generating first data on a first shape for an intraoral appliance for the one or more target teeth, based on the at least one image; generating second data on a second shape of the intraoral appliance, based on data on one or more antagonist teeth corresponding to the one or more target teeth and the first data; and generating third data on the intraoral appliance in which the first shape and the second shape are combined, based on the first data and the second data.

According to one embodiment of the present disclosure, the generating the first data comprises: generating data on an inner surface of the first shape, based on a shape of the one or more target teeth; generating data on a contour of the first shape, based on one or more contour points and one or more connection points for the one or more target teeth; and generating data on an outer surface of the first shape, based on the data on the inner surface and the data on the contour.

According to one embodiment of the present disclosure, the generating the first data comprises: determining a first contour point for a first target tooth among the one or more target teeth in a first manner, based on the at least one image; and determining a second contour point for a second target tooth among the one or more target teeth in a second manner different from the first manner.

According to one embodiment of the present disclosure, the one or more target teeth are four anterior teeth, the first target tooth is one of inner teeth among the four anterior teeth, and the second target tooth is one of outer teeth among the four anterior teeth.

According to one embodiment of the present disclosure, the determining the first contour point in the first manner comprises: setting an x-coordinate value of the first contour point to an x-coordinate value of a center of a bounding box for the first target tooth; and setting a y-coordinate value of the first contour point to a y-coordinate value of a reference line parallel to an occlusal surface of the oral cavity.

According to one embodiment of the present disclosure, the determining the second contour point in the second manner comprises determining the second contour point, based on an intersection between a straight line connecting a center of the oral cavity and a center of a bounding box for the second target tooth and a surface of the outer teeth.

According to one embodiment of the present disclosure, the determining the second contour point in the second manner comprises: determining a first point that is spaced apart from the center of the oral cavity in a y-axis direction by a value obtained by adding a predetermined value to a y-coordinate value of a reference line parallel to an occlusal surface of the oral cavity; determining a second point that is spaced apart from a point existing within a bounding box for the second target tooth in the y-axis direction by a value obtained by adding a predetermined value to the y-coordinate value of the reference line parallel to the occlusal surface of the oral cavity; determining a first intersection between a straight line connecting the first point and the second point and a surface of the second target tooth; determining a third point that is spaced apart from the second point in an x-axis direction by a predetermined distance;

determining a second intersection between a straight line connecting the first point and the third point and the surface of the second target tooth; and setting the second contour point to a farther one of the first intersection and the second intersection from the first point.

According to one embodiment of the present disclosure, the predetermined value represents movement of the second target tooth in a direction from the root toward the crown.

According to one embodiment of the present disclosure, the generating the first data comprises: determining a reference point that is spaced a predetermined distance apart from a center of a bounding box for the second target tooth in a direction away from a center of the oral cavity; and determining a point on a surface of the second target tooth, which is closest to the reference point, as a connection point.

According to one embodiment of the present disclosure, the generating the second data comprises: determining a width indicating an x-axis length of the second shape; determining a height indicating a y-axis length of the second shape; and determining a depth indicating a z-axis length of the second shape.

According to one embodiment of the present disclosure, the determining the width of the second shape comprises determining the width of the second shape, based on an x-axis length of the one or more target teeth.

According to one embodiment of the present disclosure, the determining the height of the second shape comprises: determining a length by which the second shape protrudes from the first shape in a direction toward the one or more antagonist teeth; and determining the height of the second shape, based on the protruding length.

According to one embodiment of the present disclosure, the determining the depth of the second shape comprises: determining a first depth range of a portion of an outer surface of the first shape where the second shape is to be combined, the portion being within a predetermined height from the highest point; determining a second depth range of a portion of a surface of the one or more antagonist teeth that is to contact the second shape when the intraoral appliance is mounted in the oral cavity, the portion being within a predetermined height from the highest point; and determining the depth of the second shape, based on the first depth range and the second depth range.

According to one embodiment of the present disclosure, the determining the depth of the second shape comprises determining the depth of the second shape by merging the first depth range and the second depth range and then adding a predetermined offset thereto.

According to one embodiment of the present disclosure, the generating the third data comprises: in a case where the one or more target teeth are maxillary teeth, generating the third data so that a center of the second shape is positioned closer to a lingual side relative to the one or more target teeth when the intraoral appliance is mounted in the oral cavity.

According to one embodiment of the present disclosure, the generating the third data comprises: in a case where the one or more target teeth are mandibular teeth, generating the third data so that a center of the second shape is positioned closer to a labial side relative to the one or more target teeth when the intraoral appliance is mounted in the oral cavity.

According to one embodiment of the present disclosure, the intraoral appliance is an NTI splint.

In one aspect of the present disclosure, there may be provided an electronic device. An electronic device according to one aspect of the present disclosure may include: one or more processors; and one or more memories in which instructions executed by the one or more processes are stored, wherein the one or more processors are configured to, when the instructions are executed by the one or more processors, execute the method according to the present disclosure.

In one aspect of the present disclosure, there may be provided a non-transitory computer-readable recording medium. A non-transitory computer-readable recording medium according to one aspect of the present disclosure may record instructions that, when executed by one or more processors, cause the one or more processors to perform an operation, wherein the instructions may be configured to cause the one or more processors to execute a method according to the present disclosure.

### ADVANTAGEOUS EFFECTS

According to various embodiments of the present disclosure, an intraoral appliance that simultaneously considers both comfort and aesthetics can be automatically generated, and computational resources can be saved by eliminating the need for a plurality of user operations and corresponding calculations for generating and adjusting the intraoral appliance.

The effects of the technical concepts of the present disclosure are not limited to those mentioned above, and other effects not mentioned will be clearly understood by those skilled in the art from the descriptions herein.

### DESCRIPTION OF DRAWINGS

FIG. 1 is a diagram illustrating a state in which an image of a patient's oral cavity is obtained using an intraoral scanner according to an embodiment of the present disclosure.
FIG. 2A is a block diagram of an electronic device and an intraoral scanner according to an embodiment of the present disclosure. FIG. 2B is a perspective view of an intraoral scanner according to an embodiment of the present disclosure.
FIG. 3 is a diagram illustrating a method for generating a three-dimensional image 320 of an oral cavity according to an embodiment of the present disclosure.
FIG. 4 is a diagram illustrating a method for generating data on an intraoral appliance, based on an image of an oral cavity, according to an embodiment of the present disclosure.
FIG. 5 is a diagram illustrating a method for generating first data based on a three-dimensional image of an oral cavity according to an embodiment of the present disclosure.
FIG. 6 is a diagram illustrating a method for determining an x-coordinate of a second contour point in a second target tooth according to an embodiment of the present disclosure.
FIG. 7 is a diagram illustrating a method for determining a connection point according to an embodiment of the present disclosure.
FIG. 8 illustrates a second shape combined with a first shape according to an embodiment of the present disclosure.
FIG. 9 illustrates a combined form of a first shape and a second shape according to an embodiment of the present disclosure.
FIG. 10 is a diagram illustrating a method for determining a depth of a second shape according to an embodiment of the present disclosure.
FIG. 11 illustrates a state in which an intraoral appliance generated based on third data is mounted within the oral cavity according to an embodiment of the present disclosure.
FIG. 12 illustrates a state in which an intraoral appliance generated based on the maxilla is mounted within the oral cavity according to an embodiment of the present disclosure.
FIG. 13 illustrates a state in which an intraoral appliance generated based on the mandible is mounted within the oral cavity according to an embodiment of the present disclosure.
FIG. 14 is a flowchart of a method for generating an intraoral appliance according to an embodiment of the present disclosure.

### DETAILED DESCRIPTION

The various embodiments described in this document are presented for the purpose of clearly explaining the technical concept of the present disclosure, and are not intended to limit the present disclosure to any particular embodiment. The technical concept of the present disclosure includes various modifications, equivalents, and alternatives of the respective embodiments described in the present document, as well as embodiments selectively combined from all or some of the respective embodiments. In addition, the scope of the technical concept of the present disclosure is not limited to the various embodiments presented below or the specific descriptions thereof.

Unless otherwise defined, the terms used in this document, including technical or scientific terms, may have meanings that are generally understood by those skilled in the art to which the present disclosure pertains.

Expressions such as "include," "may include," "provided," "may be provided," "have," and "may have" used herein indicate the presence of the corresponding features (e.g., functions, operations, components, etc.) and do not preclude the presence of additional features. That is, such expressions are to be understood as open-ended terms that imply the possibility of including other embodiments.

Singular forms used in this document may include plural forms unless the context clearly indicates otherwise, and this likewise applies to singular expressions recited in the claims.

Expressions such as "first," "second," or "primary," "secondary," and the like, used in this document, are employed merely to distinguish one element from another among a plurality of elements, unless the context clearly indicates otherwise, and do not limit the order or importance of the elements.

Expressions such as "A, B, and C," "A, B, or C," "A, B, and/or C," "at least one of A, B, and C," "at least one of A, B, or C," "at least one of A, B, and/or C," as used in the present document, may refer to each of the listed items or any possible combination of the listed items. For example, the expression "at least one of A or B" may refer to all of (1) at least one A, (2) at least one B, and (3) at least one A and at least one B.

The term "unit" used in this document refers to a software component or hardware component, such as a field-programmable gate array (FPGA) and an application specific integrated circuit (ASIC). However, a "unit" is not limited to software and hardware. A "unit" may be configured to be an addressable storage medium or may be configured to run on one or more processors. In an embodiment, a "unit" may include components, such as software components, object-oriented software components, class components, and task components, as well as processors, functions, attributes, procedures, subroutines, segments of program codes, drivers, firmware, micro-codes, circuits, data, databases, data structures, tables, arrays, and variables.

The expression "based on" used herein is used to describe one or more factors that influences a decision, an action of judgment or an operation described in a phrase or sentence including the relevant expression, and this expression does not exclude additional factor influencing the decision, the action of judgment or the operation.

The expressions "one component (e.g., a first component) is 'connected' or 'coupled' to another component (e.g., a second component)," as used in this document, may refer not only to the first component being directly connected or coupled to the second component, but also to the first component being connected or coupled to the second component indirectly through another component (e.g., a third component).

The expression "configured to," as used in this document, may have meanings such as "set to," "capable of," "modified to," "designed to," or "able to," depending on the context. The expression is not limited to the meaning "specifically hardware-designed," and, for example, a processor configured to perform a specific operation may refer to a generic-purpose processor capable of performing the specific operation by executing software.

Hereinafter, various embodiments of the present disclosure will be described with reference to the accompanying drawings. In the accompanying drawings and the descriptions thereof, the same or substantially equivalent components may be designated by the same reference numerals. In addition, in the following descriptions of the various embodiments, repetitive descriptions of identical or corresponding components may be omitted, but this does not mean that such components are not included in the embodiments.

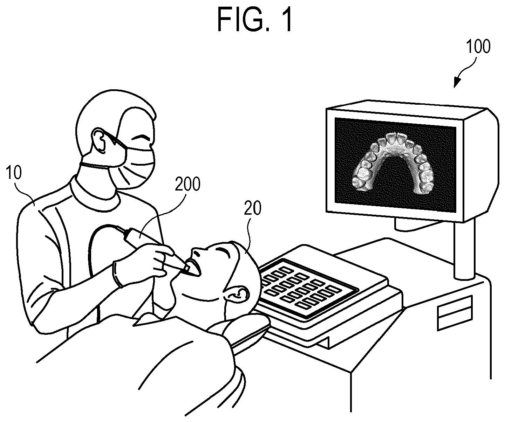

FIG. 1 is a diagram illustrating a state in which an image of a patient's oral cavity is obtained using a three-dimensional scanner 200 according to an embodiment of the present disclosure. According to an embodiment, the three-dimensional scanner 200 may be a dental medical device for obtaining an image of an oral cavity of a subject 20. For example, the three-dimensional scanner 200 may be an intraoral scanner. As illustrated in FIG. 1, a user 10 (e.g., a dentist or dental hygienist) may obtain an image of the oral cavity of the subject 20 (e.g., a patient) using the three-dimensional scanner 200. As another example, the user 10 may obtain an image of the oral cavity of the subject 20 from a diagnostic model (e.g., a plaster model or impression model) modeling the shape of the oral cavity of the subject 20. For convenience of explanation, the following description describes obtaining an image of the oral cavity of the subject 20 by scanning the oral cavity of the subject 20, but the present disclosure is not limited thereto, and it is also possible to obtain an image of other regions of the subject 20 (e.g., an ear of the subject 20). The three-dimensional scanner 200 may be a handheld scanner that may be inserted into and removed from the oral cavity and that has a scanning distance and scanning angle freely adjustable by the user 10.

The three-dimensional scanner 200 according to an embodiment may be inserted into the oral cavity of the subject 20 and may scan the oral cavity in a non-contact manner, thereby obtaining an image of the oral cavity. The image of the oral cavity may include at least one tooth or gingiva. The three-dimensional scanner 200 may irradiate light into the oral cavity of the subject 20 (e.g., at least one tooth or gingiva of the subject 20) using a light source (or projector) and receive light reflected from the oral cavity of the subject 20 through a camera (or at least one image sensor). According to another embodiment, the three-dimensional scanner 200 may obtain an image of a diagnostic model of the oral cavity by scanning the diagnostic model of the oral cavity. If the diagnostic model of the oral cavity is a diagnostic model modeled after the oral cavity of the subject 20, the image of the diagnostic model of the oral cavity may be the image of the oral cavity of the subject. For convenience of explanation, the following description assumes that an image of the oral cavity is obtained by scanning the oral cavity of the subject 20, but the present disclosure is not limited thereto.

The three-dimensional scanner 200 according to an embodiment may obtain a surface image of the oral cavity of the subject 20 as a two-dimensional image, based on information received via the camera. The surface image of the oral cavity of the subject 20 may include at least one of at least one tooth, gingiva, artificial structure, or the cheek, tongue, or lips of the subject 20. The surface image of the oral cavity of the subject 20 may be a two-dimensional image.

The two-dimensional image of the oral cavity obtained by the three-dimensional scanner 200 according to an embodiment may be transmitted to an electronic device 100 connected via a wired or wireless communication network. The electronic device 100 may be a computer device or a portable communication device. The electronic device 100 may generate a three-dimensional image of the oral cavity (also referred to as a three-dimensional oral cavity image, a three-dimensional oral cavity model, a three-dimensional scan model, a teeth model, a three-dimensional oral cavity representation, a three-dimensional numerical entity, etc.), representing the oral cavity in three dimensions, based on a two-dimensional image of the oral cavity received from the three-dimensional scanner 200. The electronic device 100 may generate a three-dimensional image of the oral cavity by three-dimensionally modeling the internal structure of the oral cavity, based on the received two-dimensional image of the oral cavity.

The three-dimensional image may include not only a subject's geometric information within a three-dimensional space, but also information such as color, texture, and material. The format of the three-dimensional image may be, for example, one of a standard triangle language (STL), OBJ, or polygon file, but is not limited thereto. "Polygon" may refer to the smallest unit of a polygon used to represent the three-dimensional shape of a three-dimensional oral cavity model. For example, the surface of the three-dimensional image may be represented by triangular polygons. For example, a polygon may include at least three vertices and one face. The three-dimensional image may include information about each vertex, such as its position in three-dimensional space, color, and normal. A mesh is made up of a plurality of polygons and may represent the geometry of a subject within a three-dimensional space. As the number of polygons representing the three-dimensional image increases, the subject may be expressed in greater detail.

A three-dimensional scanner 200 according to another embodiment may scan the oral cavity of a subject 20 to obtain a two-dimensional image of the oral cavity, generate a three-dimensional image of the oral cavity on the basis of the obtained two-dimensional image of the oral cavity, and transmit the generated three-dimensional image of the oral cavity to the electronic device 100.

The electronic device 100 according to an embodiment may be communicatively connected to a cloud server (not shown). In the above case, the electronic device 100 may transmit the two-dimensional image of the oral cavity of the subject 20 or the three-dimensional image of the oral cavity to the cloud server, and the cloud server may store the two-dimensional image of the oral cavity of the subject 20 or the three-dimensional image of the oral cavity, which is received from the electronic device 100.

According to another embodiment, the three-dimensional scanner may be a table scanner (not shown) that is fixed to a specific location, in addition to a handheld scanner that is inserted into the oral cavity of the subject 20. The table scanner may generate a three-dimensional image of a diagnostic model of the oral cavity by scanning the diagnostic model. In the above case, since the light source (or projector) and camera of the table scanner are fixed, the user may scan the diagnostic model of the oral cavity while moving the arm that holds the diagnostic model of the oral cavity. Compared to handheld scanners, although table scanners are less likely to cause noise due to other objects interfering with the camera and the diagnostic model during scanning, the embodiments of the present disclosure are applicable not only to handheld scanners but also to table scanners and other three-dimensional scanners.

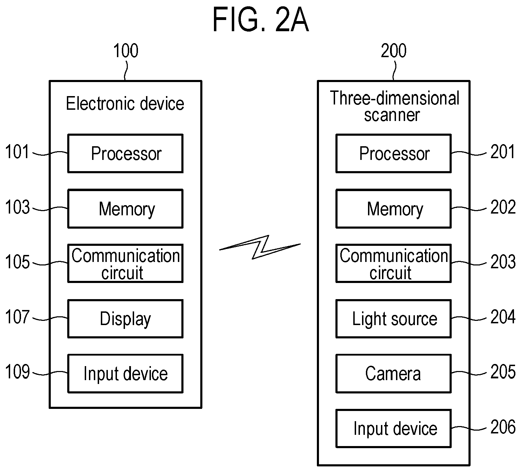

FIG. 2A is a block diagram of an electronic device 100 and a three-dimensional scanner 200 according to an embodiment of the present disclosure. The electronic device 100 and the three-dimensional scanner 200 may be connected to each other via a wired or wireless communication network and may exchange various data.

The three-dimensional scanner 200 according to an embodiment may include a processor 201, a memory 202, a communication circuit 203, a light source 204, a camera 205, an input device 206, and/or a sensor module 207. At least one of the components included in the three-dimensional scanner 200 may be excluded, or other components may be added to the three-dimensional scanner 200. Additionally or alternatively, some of the components may be implemented into an integrated form or implemented as single or multiple entities. At least some of the components within the three-dimensional scanner 200 may be interconnected via a bus, a general-purpose input/output (GPIO), a serial peripheral interface (SPI), or a mobile industry processor interface (MIPI), thereby exchanging data and/or signals.

The processor 201 of the three-dimensional scanner 200 according to an embodiment is a component capable of performing operations or data processing related to the control or communication of the respective components of the three-dimensional scanner 200, and may be operatively connected to the components of the three-dimensional scanner 200. The processor 201 may load commands or data received from other components of the three-dimensional scanner 200 into the memory 202, process the commands or data stored in the memory 202, and store the resulting data. The memory 202 of the three-dimensional scanner 200 according to an embodiment may store instructions for the operation of the processor 201 described above.

According to an embodiment, the communication circuit 203 of the three-dimensional scanner 200 may establish a wired or wireless communication channel with an external device (e.g., the electronic device 100), and transmit and receive various data to and from the external device. According to an embodiment, the communication circuit 203 may include at least one port for connecting to the external device via a wired cable to enable wired communication with the external device. In this case, the communication circuit 203 may communicate with the external device in a wired connection state through at least one port. According to an embodiment, the communication circuit 203 may include a cellular communication module and may be configured to connect to a cellular network (e.g., 3G, LTE, 5G, WiBro, or WiMAX). According to an embodiment, the communication circuit 203 may include a short-range communication module to transmit and receive data to and from an external device using short-range communication (e.g., Wi-Fi, Bluetooth, Bluetooth Low Energy (BLE), or UWB), but the present disclosure is not limited thereto. According to an embodiment, the communication circuit 203 may include a contactless communication module for contactless communication. For example, the contactless communication may include at least one contactless proximity communication technology, such as near-field communication (NFC), radio frequency identification (RFID) communication, or magnetic secure transmission (MST) communication.

The light source 204 of the three-dimensional scanner 200 according to an embodiment may irradiate light toward the oral cavity of the subject 20. For example, the light emitted from the light source 204 may be structured light having a predetermined pattern (e.g., a stripe pattern in which linear patterns of different colors appear continuously). The structured light pattern may be generated using a pattern mask or a digital micro-mirror device (DMD), but is not limited thereto. The camera 205 of the three-dimensional scanner 200 according to an embodiment may obtain an image regarding the oral cavity of the subject 20 by receiving light reflected by the oral cavity of the subject 20. The camera 205 may include a left camera corresponding to the left-eye field of view and a right camera corresponding to the right-eye field of view to construct three-dimensional images according to an optical triangulation method. The camera 205 may include at least one image sensor, such as a CCD sensor or a CMOS sensor.

The input device 206 of the three-dimensional scanner 200 according to an embodiment may receive user input for controlling the three-dimensional scanner 200. The input device 206 may include a button for receiving a push operation from the user 10, a touch panel for detecting a touch of the user 10, and a voice recognition device including a microphone. For example, the user 10 may control starting or stopping of scanning using the input device 206.

The sensor module 207 of the three-dimensional scanner 200 according to an embodiment may detect an operating state of the three-dimensional scanner 200 or an external environmental state (e.g., a user's operation) and generate an electrical signal corresponding to the detected state. For example, the sensor module 207 may include at least one of a gyro sensor, an acceleration sensor, a gesture sensor, a proximity sensor, or an infrared sensor. The user 10 may control the start or stop of scanning using the sensor module 207. For example, in a case where the user 10 holds and moves the three-dimensional scanner 200, the processor 201 may control the three-dimensional scanner 200 to start a scanning operation when the angular velocity measured by the sensor module 207 exceeds a predetermined threshold value.

According to an embodiment, the three-dimensional scanner 200 may start scanning in response to receiving a user input to start scanning through the input device 206 of the three-dimensional scanner 200 or the input device 109 of the electronic device 100, or in response to processing in the processor 201 of the three-dimensional scanner 200 or the processor 101 of the electronic device 100. When the user 10 scans the oral cavity of the subject 20 through the three-dimensional scanner 200, the three-dimensional scanner 200 may generate a two-dimensional image of the oral cavity of the subject 20 and transmit the two-dimensional image of the oral cavity of the subject 20 to the electronic device 100 in real time. The electronic device 100 may display the received two-dimensional image of the oral cavity of the subject 20 through the display 107. In addition, the electronic device 100 may generate (construct) a three-dimensional image of the oral cavity of the subject 20, based on the two-dimensional image of the oral cavity of the subject 20, and display, through the display 107, the three-dimensional image of the oral cavity. The electronic device 100 may also display the three-dimensional image being generated in real time through the display 107.

The electronic device 100 according to an embodiment may include one or more processors 101, one or more memories 103, a communication circuit 105, a display 107, and/or an input device 109. At least one of the components included in the electronic device 100 may be excluded, or other components may be added to the electronic device 100. Additionally or alternatively, some components may be implemented in an integrated form, or implemented as a single or multiple entities. At least some components within the electronic device 100 may be connected to each other via a bus, a general-purpose input/output (GPIO), a serial peripheral interface (SPI), or a mobile industry processor interface (MIPI), and may exchange data and/or signals.

According to an embodiment, one or more processors 101 of the electronic device 100 may be configured to perform operations or data processing related to control and/or communication of the respective components (e.g., the memory 103) of the electronic device 100. One or more processors 101 may be operatively connected to, for example, the components of the electronic device 100. One or more processors 101 may load commands or data received from other components of the electronic device 100 into one or more memories 103, process the commands or data stored in one or more memories 103, and store resulting data.

According to an embodiment, one or more memories 103 of the electronic device 100 may store instructions for the operations of the one or more processors 101. One or more memories 103 may store data received from the three-dimensional scanner 200 (e.g., a two-dimensional image of the oral cavity obtained through an oral cavity scan).

According to an embodiment, the communication circuit 105 of the electronic device 100 may establish a wired or wireless communication channel with an external device (e.g., the three-dimensional scanner 200 or a cloud server), and transmit and receive various data to and from the external device. According to an embodiment, the communication circuit 105 may include at least one port for connecting to the external device via a wired cable in order to perform wired communication with the external device. In this case, the communication circuit 105 may communicate with the external device in a wired connection state through at least one port. According to an embodiment, the communication circuit 105 may be configured to include a cellular communication module so as to connect to a cellular network (e.g., 3G, LTE, 5G, WiBro, or WiMAX). According to an embodiment, the communication circuit 105 may include a short-range communication module to transmit and receive data to and from an external device using short-range communication (e.g., Wi-Fi, Bluetooth, Bluetooth Low Energy (BLE), or UWB), but the present disclosure is not limited thereto. According to an embodiment, the communication circuit 105 may include a contactless communication module for contactless communication. For example, the contactless communication may include at least one contactless proximity communication technology, such as near-field communication (NFC), radio frequency identification (RFID) communication, or magnetic secure transmission (MST) communication.

The display 107 of the electronic device 100 according to an embodiment may display various screens under the control of the processor 101. The processor 101 may display a two-dimensional image of the oral cavity of the subject 20, which is received from the three-dimensional scanner 200, and/or a three-dimensional image of the oral cavity, which is obtained by modelling the intraoral structure in three dimensions, through the display 107. For example, a two-dimensional image and/or a three-dimensional image of the oral cavity may be displayed using a specific application program. In the above case, the user 10 may edit, save, and delete two-dimensional images and/or three-dimensional images of the oral cavity.

The input device 109 of the electronic device 100 according to an embodiment may receive commands or data to be used in the components (e.g., one or more processors 101) of the electronic device 100 from the source (e.g., the user) outside the electronic device 100. The input device 109 may include, for example, a microphone, a mouse, or a keyboard. According to an embodiment, the input device 109 may be implemented in the form of a touch sensor panel that is coupled to the display 107 and capable of recognizing contact or proximity of various external objects.

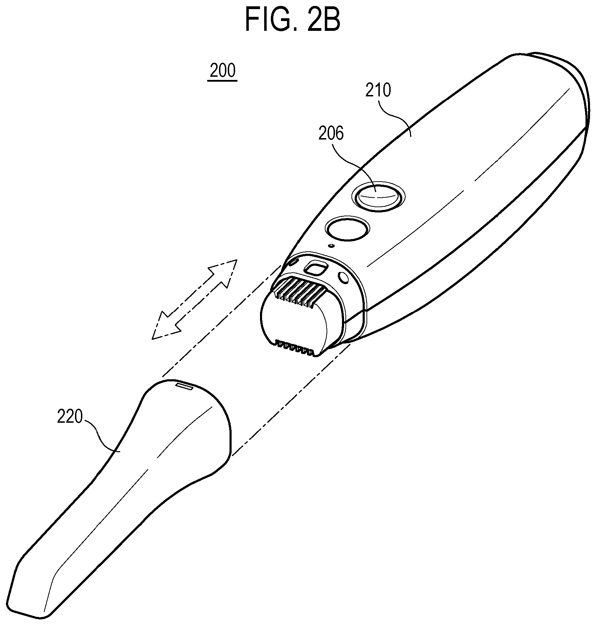

FIG. 2B is a perspective view of the three-dimensional scanner 200 according to an embodiment of the present disclosure. The three-dimensional scanner 200 according to an embodiment may include a main body 210 and a probe tip 220. The main body 210 of the three-dimensional scanner 200 may have a shape configured to be easily held with a hand of the user 10. The probe tip 220 may have a shape configured to be easily inserted into and removed from the oral cavity of the subject 20. In addition, the main body 210 may be coupled to and separated from the probe tip 220. The components of the three-dimensional scanner 200 described in FIG. 2A may be provided within the main body 210. An opening may be formed at one end of the main body 210 to allow light output from the light source 204 to be irradiated onto the subject 20. Light irradiated through the opening may be reflected by the subject 20 and then re-enter through the opening. The reflected light entering through the opening may be captured by the camera 205 to generate an image of the subject 20. The user 10 may initiate a scan using the input device 206 (e.g., a button) of the three-dimensional scanner 200. For example, when the user 10 touches or presses the input device 206, light may be irradiated from the light source 204 onto the subject 20.

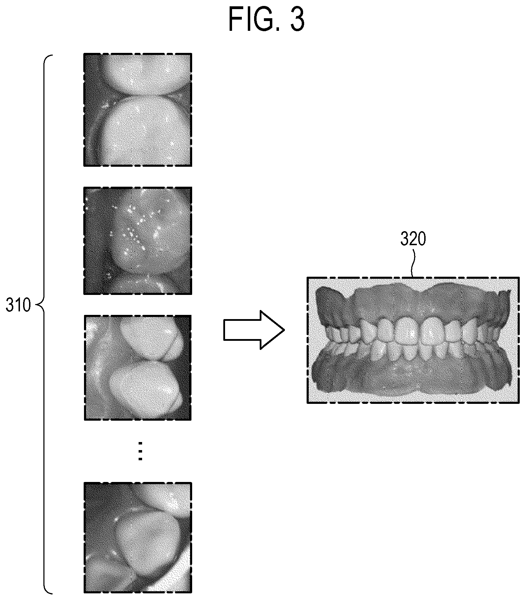

FIG. 3 is a diagram illustrating a method for generating a three-dimensional image 320 of an oral cavity according to an embodiment of the present disclosure. The user 10 may scan the oral cavity of the subject 20 while moving the three-dimensional scanner 200. In this case, the three-dimensional scanner 200 may obtain a plurality of two-dimensional images 310 of the oral cavity of the subject 20. For example, the three-dimensional scanner 200 may obtain a two-dimensional image of a region including the front teeth of the subject 20, a two-dimensional image of a region including the molars of the subject 20, and the like. The three-dimensional scanner 200 may transmit the plurality of obtained two-dimensional images 310 to the electronic device 100. According to another embodiment, the user 10 may scan a diagnostic model of the oral cavity while moving the three-dimensional scanner 200, thereby obtaining a plurality of two-dimensional images of the diagnostic model of the oral cavity. For convenience of explanation, the following description assumes that an image of the oral cavity of the subject 20 is obtained by scanning the oral cavity, but the present disclosure is not limited thereto.

The electronic device 100 according to an embodiment may convert each of the plurality of two-dimensional images 310 of the oral cavity of the subject 20 into a set of a plurality of points having three-dimensional coordinate values. For example, the electronic device 100 may convert each of the plurality of two-dimensional images 310 into a point cloud, which is a set of data points with three-dimensional coordinate values. For example, a point cloud set, which is a set of three-dimensional coordinate values based on the plurality of two-dimensional images 310, may be stored as raw data for the oral cavity of the subject 20. The electronic device 100 may complete the entire tooth model by aligning the point clouds, which are sets of data points having three-dimensional coordinate values.

The electronic device 100 according to an embodiment may reconfigure (reconstruct) a three-dimensional image of the oral cavity. For example, the electronic device 100 may reconfigure a three-dimensional image 320 of the oral cavity of the subject 20 by reconstructing a plurality of points through merging a set of point clouds stored as raw data using a Poisson algorithm and converting the reconstructed points into a closed three-dimensional surface.

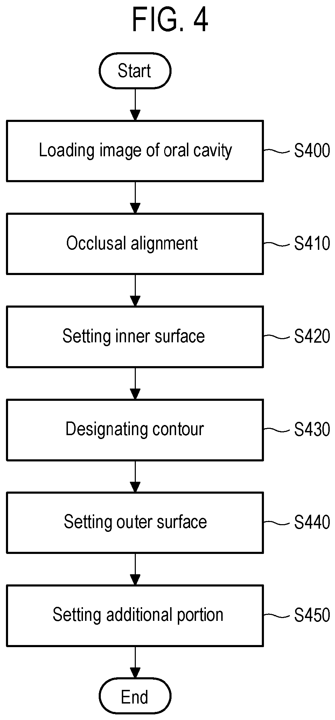

FIG. 4 is a diagram illustrating a method for generating data on an intraoral appliance on the basis of an image of an oral cavity according to an embodiment of the present disclosure.

An electronic device 100 according to an embodiment of the present disclosure may load an image of an oral cavity (S400). In an embodiment, the electronic device 100 may load an image 320 of the oral cavity generated based on an image received from the three-dimensional scanner 200 via the communication circuit 105. The electronic device 100 may process the received image or load an image 320 of the oral cavity pre-stored in the processor 101 or memory 103 and display it on the display 107.

The electronic device 100 according to an embodiment of the present disclosure may analyze and align the shape of the received image 320 of the oral cavity (S410). In an embodiment, the electronic device 100 may automatically set an occlusal plane for the image 320 of the oral cavity, align the image 320 of the oral cavity according to the occlusal plane, and then display the image 320 of the oral cavity through the display 107. Alternatively or additionally, the electronic device 100 may set an occlusal plane for the image 320 of the oral cavity, based on an input for the image 320 of the oral cavity (e.g., an input designating a reference point), and align the image 320 of the oral cavity along the set occlusal plane.

According to an embodiment of the present disclosure, the electronic device 100 may set an inner surface of an intraoral appliance for the aligned image 320 of the oral cavity (S420). Here, the intraoral appliance may refer to a structure inserted and mounted in the oral cavity to treat temporomandibular joint disorders, tooth trauma, cavities, gum disease, etc., and may include splints, crowns, inlays, onlays, copings, pontics, and veneers, but the technical concept of the present disclosure is not limited to these examples. Here, the intraoral appliance may be a fixture (device) designed for various purposes, such as patient's tooth fixing, orthodontic treatment, or tooth protection, and may be mounted within the oral cavity of the patient. For example, the intraoral appliance may be referred to as a tooth fixation device for fixing a patient's teeth, a tooth orthodontic device for correcting a patient's teeth, or a tooth protection device for protecting a patient's teeth. For example, the intraoral appliance may include a splint. Meanwhile, the present disclosure is not limited thereto, and the intraoral appliance is a term encompassing various types of devices that may be mounted within the oral cavity. Even if referred to by terms having the same or similar meaning, the embodiments of the present disclosure may be applied to such devices.

The electronic device 100 according to an embodiment of the present disclosure may set the inner surface of an intraoral appliance. In an embodiment, the electronic device 100 may set the inner surface of the intraoral appliance, based on an inner surface offset distance, surface smoothness, etc. input from the input device 109 or preset. Here, the inner surface offset distance may refer to a normal distance between the image 320 of the oral cavity and the inner surface of the intraoral appliance, and the surface smoothness may refer to roughness of the inner surface of the intraoral appliance.

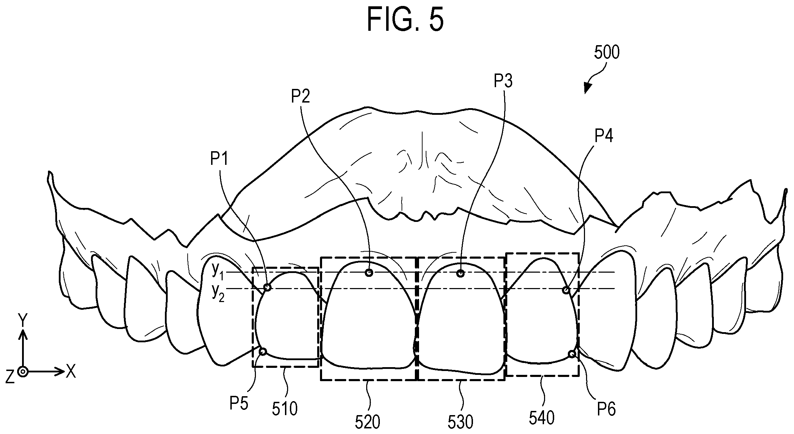

The electronic device 100 according to an embodiment of the present disclosure may designate the contour of the intraoral appliance, based on the image 320 of the oral cavity (S430). In an embodiment, the electronic device 100 may designate the contour of the intraoral appliance, based on a buccal height, a lingual height, etc. input from the input device 109 or preset. For illustrative purposes only, the buccal height of the intraoral appliance may be the height of the outer tooth wall facing the cheek relative to the lower surface of the teeth region. As a more specific example, the buccal height may be the height formed along the outer tooth wall relative to the bottom surface of the maxillary teeth region. In this case, as the buccal height increases, the buccal contour formed may be positioned closer to the gingiva. As another example for understanding, the lingual height may be the height of the inner tooth wall facing the tongue relative to the lower surface of the teeth region. As a more specific example, the lingual height may be the height formed along the inner tooth wall relative to the bottom surface of the maxillary teeth region. In this case, as the lingual height increases, the lingual contour formed may be positioned closer to the gingiva. A method for designating the contour of an intraoral appliance according to various embodiments of the present disclosure will be described in detail with reference to FIGS. 5 to 7.

The electronic device 100 according to an embodiment of the present disclosure may set the outer surface of the intraoral appliance, based on the image 320 of the oral cavity (S440). The electronic device 100 may set the outer surface of the intraoral appliance, based on a thickness, surface smoothness, etc. input from the input device 109 or preset. In this case, the thickness may be a maximum thickness of the intraoral appliance extending in the occlusal direction (e.g., a thickness from the inner surface of the intraoral appliance in the buccal/lingual direction). The surface smoothness may refer to roughness of the outer surface of the intraoral appliance.

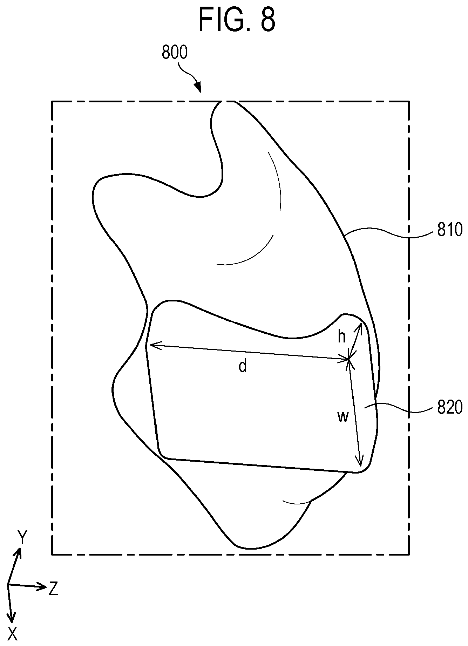

The electronic device 100 according to an embodiment of the present disclosure may set an additional portion of the intraoral appliance (S450). A method for setting an additional portion of the intraoral appliance according to various embodiments of the present disclosure will be described in detail with reference to FIGS. 8 to 10.

The electronic device 100 according to an embodiment of the present disclosure may generate data on the intraoral appliance, based on the inner surface, outer surface, and additional portions of the intraoral appliance determined through the above process. The generated data on the intraoral appliance may be transmitted to an external device via the communication circuit 105 and output as the intraoral appliance. The external device may be a 3D printer, but is not limited thereto.

The electronic device 100 according to an embodiment of the present disclosure may utilize a neural network model to perform at least some of the above steps. For example, the electronic device 100 may perform an inference operation related to the data on the intraoral appliance appropriate for the loaded image 320 of the oral cavity using a neural network model trained based on three-dimensional images of a plurality of oral cavities and corresponding data on a plurality of intraoral appliances.

The electronic device 100 according to an embodiment of the present disclosure may receive an input regarding an occlusion state between the maxillary image and the mandibular image or a minimum distance to antagonist within the image 320 of the oral cavity and, based on the input, adjust the occlusion state or the minimum distance to antagonist in each of the steps S420 to S450. During this adjustment operation, the electronic device 100 may perform a calculation operation on the image 320 of the oral cavity, thereby displaying the occlusion state, the minimum distance to antagonist, or the like.

FIG. 5 is a diagram illustrating a method for generating first data on the basis of a three-dimensional image of an oral cavity according to an embodiment of the present disclosure.

To explain various embodiments of the present disclosure, an orthogonal coordinate system having an x-axis, a y-axis, and a z-axis, which are orthogonal to each other, may be defined. As used herein, the expressions "x-axis direction," "y-axis direction," and "z-axis direction" of the orthogonal coordinate system may refer to both directions along which each axis of the orthogonal coordinate system extends, unless specifically defined otherwise in the description. Furthermore, a + sign preceding each axis direction may indicate a positive direction, which is one of the two directions extending along the corresponding axis, and a - sign preceding each axis direction may indicate a negative direction, which is the other of the two directions extending along the corresponding axis. For example, in the three-dimensional image of the oral cavity 500 in FIG. 5, the x-axis direction may refer to a horizontal direction, the y-axis direction may refer to a vertical direction, and the z-axis direction may refer to a direction exiting or entering the screen. Furthermore, "x-coordinate," "y-coordinate," and "z-coordinate" may indicate a location in the x-axis direction, a location in the y-axis direction, and a location in the z-axis direction, respectively, and "width," "height," and "depth" may indicate an x-axis length, a y-axis length, and a z-axis length of an object, respectively. Furthermore, "height" may be measured in a direction from the root of each tooth toward the crown. That is, the directions in which the "height" is measured in the maxilla and mandible may be opposite to each other.

The electronic device 100 according to an embodiment of the present disclosure may set target teeth. Target teeth may refer to one or more teeth on which an intraoral appliance is mounted and which serve as references for generating the inner surface of the intraoral appliance. According to an embodiment, the target teeth may be one or more adjacent teeth located in either the maxilla or mandible. For example, the target teeth may refer to four anterior teeth that are adjacent to each other, but the target teeth are not limited thereto. For example, the target teeth may be a set of non-adjacent teeth. According to an embodiment, the target teeth may include first target teeth, which are inner teeth among the adjacent teeth, and second target teeth, which are outer teeth among the target teeth. For example, if the target teeth are four anterior teeth, the first target teeth may refer to the two middle teeth, and the second target teeth may refer to the two teeth located at the right and left ends. Hereinafter, for convenience of explanation, the four target teeth illustrated in FIG. 5 will be referred to as a first tooth, a second tooth, a third tooth, and a fourth tooth, from left to right. According to an embodiment, the second tooth and the third tooth may be first target teeth, and the first tooth and the fourth tooth may be second target teeth.

The electronic device 100 according to an embodiment of the present disclosure may generate bounding boxes 510, 520, 530, and 540 for respective teeth. The bounding box has a cuboid shape indicating a boundary of a three-dimensional region defined within the oral cavity 500, but it is illustrated as a two-dimensional rectangle for convenience in FIG. 5. For example, the electronic device 100 may generate a first bounding box 510 for the first tooth, a second bounding box 520 for the second tooth adjacent to the first tooth, a third bounding box 530 for the third tooth adjacent to the second tooth, and a fourth bounding box 540 for the fourth tooth adjacent to the third tooth. For example, the electronic device 100 may generate a bounding box encompassing the entire oral cavity. The first to fourth bounding boxes 510, 520, 530, and 540 may indicate the boundaries of three-dimensional regions occupied by the first to fourth teeth within the oral cavity 500.

The electronic device 100 according to an embodiment of the present disclosure may set one or more contour points for each of the first and second target teeth in order to set a contour within the oral cavity 500, which serves as the basis for forming the inner surface of the intraoral appliance. Hereinafter, a method for setting one or more contour points for the target teeth will be described in detail. The electronic device 100 may set lingual contour points and buccal contour points in the same manner or in different manners.

The contour point refers to one or more points for setting the contour of the inner surface of the intraoral appliance. The contour points may include first contour points P2 and P3 for the first target teeth, second contour points P1 and P4 for the second target teeth, and connection points P5 and P6 for smoothly connecting the lingual contour points and the buccal contour points. The electronic device 100 may determine the first contour points P2 and P3 for the first target teeth in a first manner, and determine the second contour points P1 and P4 for the second target teeth in a second manner different from the first manner. Hereinafter, a method for setting the first contour points P2 and P3, the second contour points P1 and P4, and the connection points P5 and P6 will be sequentially described.

In order to set the contour points, the electronic device 100 may set a reference line having a predetermined height y1. The reference line may be a straight line parallel to the occlusal plane and spaced a predetermined distance from the occlusal plane in the root direction of the target teeth, and may be used to set the y-coordinate of the contour point.

The electronic device may set the first contour points P2 and P3 of the first target teeth in a first manner. The electronic device 100 may set the x-coordinates of the first contour points P2 and P3, based on the bounding boxes 520 and 530 of the first target teeth. The electronic device 100 may determine the first contour points P2 and P3, based on a straight line connecting the center of the oral cavity (e.g., the center of the bounding box of the entire oral cavity) and the centers of the bounding boxes 520 and 530 of the first target teeth. For example, the electronic device 100 may move both the center of the oral cavity and the centers of the bounding boxes 520 and 530 of the first target teeth in the y-axis direction by the height y1 of the reference line, and set a straight line connecting the moved points. The electronic device 100 may determine an intersection of the straight line and the first target teeth as the first contour points P2 and P3.

The first manner in which the electronic device 100 determines the first contour points P2 and P3 of the first target teeth is not limited to what was mentioned above, and the electronic device 100 may set the first contour points P2 and P3 in various ways. For example, the electronic device 100 may set the x-coordinates of the first contour points P2 and P3, based on the bounding boxes 520 and 530 of the first target teeth. The electronic device 100 may set the x-coordinates of the first contour points P2 and P3, based on the x-coordinate of the center of the bounding box. For example, the electronic device 100 may determine the x-coordinates of the centers of the bounding boxes as the x-coordinates of the first contour points P2 and P3. The electronic device 100 may set the y-coordinates of the first contour points P2 and P3, based on the reference line. For example, the electronic device 100 may determine the y-coordinate y1 of the reference line as the y-coordinates of the first contour points P2 and P3.

The electronic device 100 may set the first contour point P2 and P3 corresponding to the respective teeth of the first target teeth in the first manner described above. For example, referring to FIG. 5, the electronic device 100 may set the contour point P2 for the second tooth and the contour point P3 for the third tooth in the first target teeth.

The electronic device 100 according to an embodiment of the present disclosure may set the second contour points P1 and P4 of the second target teeth in a second manner. According to an embodiment, the electronic device 100 may determine a center of the oral cavity 500, based on the bounding boxes of the respective teeth in the maxilla and mandible. For example, the electronic device 100 may determine a center of gravity of the bounding boxes of a plurality of teeth as the center of the oral cavity 500. For example, the electronic device 100 may set a bounding box for the entire oral cavity, including all teeth within the oral cavity 500, and determine a center of the bounding box for the entire oral cavity as the center of the oral cavity 500.

The electronic device 100 according to an embodiment of the present disclosure may set the second contour points P1 and P4, based on the center of the oral cavity 500 and the intersection of a straight line connecting points included in the bounding boxes 510 and 540 for the second target teeth and the surface of the second target teeth. For example, the electronic device 100 may set the second contour points P1 and P4 in the following manner. The electronic device 100 may determine a first point that is spaced apart from the center of the oral cavity in the y-axis direction by a value y2 obtained by adding a predetermined value to the height y1 of the reference line, and a second point that is spaced apart from a point included in the bounding boxes 510 and 540 of the second target teeth by a value y2 obtained by adding a predetermined value to the height y1 of the reference line. In this case, the predetermined value may represent movement of the second target tooth from the root toward the crown. The electronic device 100 may determine a first intersection of the straight line connecting the first point and the second point and the second target teeth. Based on the second point, the electronic device 100 may generate a second straight line connecting the first point and a third point, which are spaced a predetermined distance (e.g., 0.1 mm) apart in the x-axis direction away from the midline, and if the intersection of the second straight line and the surface of the second target teeth can be found, the electronic device 100 may determine the corresponding point as the second intersection. If the second intersection can be found, the electronic device 100 may determine a new straight line and the intersection of the straight line and the surface of the second target teeth in the same manner described above. If the intersection of the second straight line and the surface of the second target teeth cannot be found, the electronic device 100 may determine the intersection of the previous straight line and the second target teeth as the second contour points P1 and P4.

The electronic device 100 may set the second contour points P1 and P4 corresponding to respective teeth of the second target teeth in the second manner described above. For example, referring to FIG. 5, the electronic device 100 may set the contour point P1 for the first tooth and the contour point P4 for the fourth tooth, which belong to the second target teeth.

The electronic device 100 according to an embodiment of the present disclosure may set connection points P5 and P6 connecting the lingual contour point and the buccal contour point. The electronic device 100 may set the contour of the first shape of the intraoral appliance by connecting the lingual contour point and the buccal contour point, and further set connection points P5 and P6 to smoothly connect the lingual contour point and the buccal contour point. For example, the electronic device 100 may set the connection points P5 and P6 using the following method. The electronic device 100 may determine a reference point spaced a predetermined distance apart from the centers of the bounding boxes 510 and 540 for the second target teeth in the direction away from the center of the oral cavity 500. For example, the electronic device 100 may set a reference point at a position spaced a distance equal to the width and height of the bounding box from the center of the bounding box in the direction away from the center of the oral cavity 500. The electronic device 100 may determine the point on the surface of the second target teeth, which is closest to the set reference point, as the connection point P5 or P6.

The electronic device 100 may set the connection points P5 and P6 in the manner described above. For example, referring to FIG. 5, the electronic device 100 may set the connection point P5 for the first tooth and a connection point P6 for the fourth tooth, which belong to the second target teeth.

The electronic device 100 may set one or more first contour points P2 and P3, second contour points P1 and P4, and connection points P5 and P6 in the manner described above. The electronic device 100 may connect the first contour points P2 and P3, the second contour points P1 and P4, and the connection points P5 and P6 to generate first data for a first shape of the intraoral appliance. A method for the electronic device 100 to generate second data for a second shape for the intraoral appliance, based on data for an antagonist tooth corresponding to the target teeth and the first data, will be described in detail later with reference to FIGS. 8 to 11.

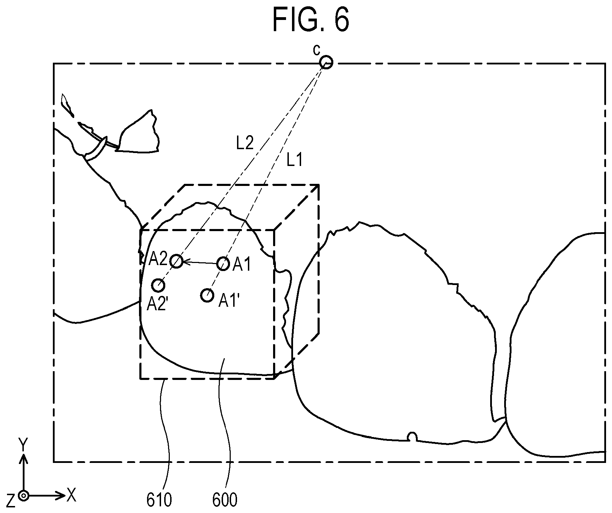

FIG. 6 is a diagram illustrating a method for determining an x-coordinate of a second contour point in second target teeth according to an embodiment of the present disclosure.

The electronic device 100 according to an embodiment of the present disclosure may determine a second contour point, based on the intersection of a straight line connecting the center of a bounding box of a given tooth and the center of the oral cavity and the tooth surface. Referring to FIG. 6, the electronic device 100 may determine a first point C spaced a predetermined value y2 apart in the y-axis from the center of the oral cavity, and generate a first straight line L1 connecting the first point C and a second point A1 spaced a predetermined value y2 apart in the y-axis from a point within a bounding box 610 of a target tooth 600. The electronic device 100 may determine a first intersection A1' of the first straight line L1 and the target tooth 600. According to an embodiment, the electronic device 100 may set a point spaced a predetermined value y2 apart in the y-axis from the center of the bounding box 610 to the second point A1. The electronic device 100 may determine a third point A2 that is spaced a predetermined distance apart from the second point A1 in the x-axis direction away from the midline, and generate a second straight line L2 connecting the third point A2 and the first point C. If the intersection of the second straight line L2 and the target tooth 600 can be found, the electronic device 100 may determine the corresponding point as the second intersection A2'. If the second intersection A2' can be found, the electronic device 100 may determine a third intersection in the same manner. For example, the electronic device 100 may determine a fourth point (not shown) that is spaced a predetermined distance apart from the third point A2 in the x-axis direction away from the midline, and determine the intersection of the third straight line (not shown) connecting the fourth point and the first point C and the target tooth 600 as the third intersection. The electronic device 100 may determine intersections in the same manner until an intersection of the straight line and the target tooth 600 cannot be found. If the second intersection A2' cannot be found, the electronic device 100 may determine the first intersection A1' as the second contour point.

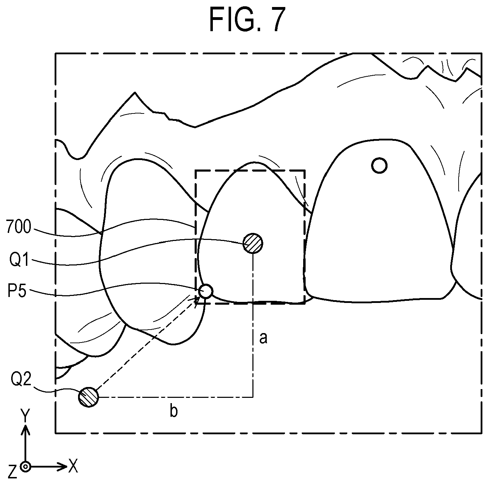

FIG. 7 is a diagram illustrating a method for determining a connection point according to an embodiment of the present disclosure.

The electronic device 100 according to an embodiment of the present disclosure may determine a connection point to smoothly connect the buccal contour point and the lingual contour point. Referring to FIG. 7, the electronic device 100 may determine the connection point using the following method. The electronic device 100 may determine a point within a bounding box 700 of a target tooth as a first reference point Q1. For example, the electronic device 100 may determine the center of the bounding box 700 as the first reference point Q1. The electronic device 100 may determine a second reference point Q2 spaced a predetermined distance apart from the first reference point Q1. For example, the electronic device 100 may determine a point spaced apart from the first reference point Q1 by a distance of "b" in the x-axis and by a distance of "a" in the y-axis as the second reference point Q2. The electronic device 100 may determine a connection point P5, based on the second reference point Q2. According to an embodiment, the electronic device 100 may determine the point closest to the second reference point Q2, from among the points located on the surface of the target teeth, as the connection point P5. According to another embodiment, the electronic device 100 may determine the point where a straight line connecting the first reference point Q1 and the second reference point Q2 meets the surface of the target teeth as the connection point P5. The method for the electronic device 100 to determine the connection point P5 is not limited to the above-described method.

FIG. 8 illustrates a second shape combined with a first shape according to an embodiment of the present disclosure.

The electronic device 100 according to an embodiment of the present disclosure may generate first data on a first shape 810 of an intraoral appliance 800, as described with reference to FIGS. 5 to 7. The electronic device 100 may generate second data on a second shape 820 for the intraoral appliance 800, based on the data for the antagonist tooth corresponding to the target tooth and the first data. The second shape 820 may be combined with the first shape 810. When the first shape 810 is generated based on the maxilla, it may be combined to the lower end of the first shape 810, and when the first shape 810 is generated based on the mandible, may be combined to the upper end of the first shape 810. According to an embodiment, when the second shape 820 is combined to the first shape 810 generated based on the maxilla, the lips may open due to the contact of the protruding portion of the second shape 820 with the lips. This openness of the lips may cause dryness in the oral cavity due to mouth breathing during sleep. Therefore, in order to prevent this, the protruding portion of the second shape 820 may be minimized when the second shape 820 is combined to the first shape 810. For example, when the first shape 810 is generated based on the maxilla, the second shape 820 may be combined to the lower end of the first shape 810. In this case, the second shape 820 may be generated to protrude posteriorly (lingually) rather than anteriorly (labially). According to another embodiment, when the second shape 820 is combined to the first shape 810 generated based on the mandible, the protruding portion of the second shape 820 may interfere with the positioning of the tongue due to lingual protrusion unrelated to the occlusion of the opposing teeth, resulting in a foreign body sensation in the device. Therefore, in order to prevent this, the second shape 820 may be generated to protrude anteriorly (labially) rather than posteriorly (lingually). For convenience of explanation, the second shape 820 will hereinafter be described as being combined to the lower end of the first shape 810. According to an embodiment, the x-axis central axis of the first shape 810 and the x-axis central axis of the second shape 820 may be the same.

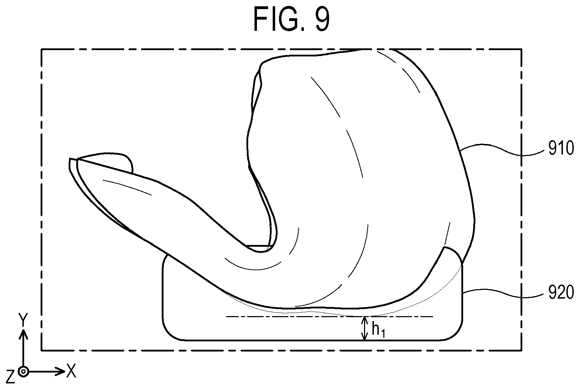

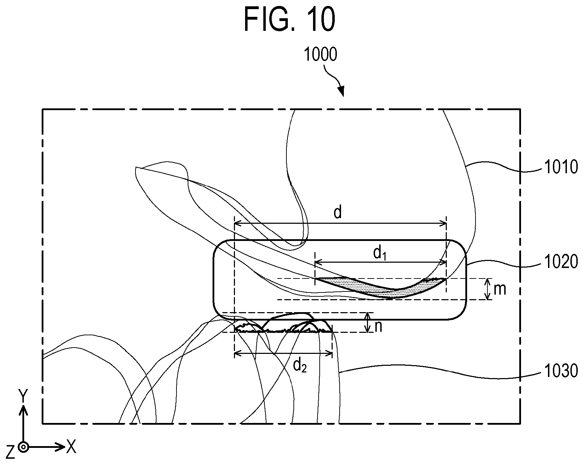

The electronic device 100 according to an embodiment of the present disclosure may determine a width w indicating an x-axis length, a height h indicating a y-axis length, and a depth d indicating a z-axis length of the second shape 820. A method for the electronic device 100 to determine the height h and depth d of the second shape 820 will be described in detail with reference to FIGS. 9 and 10, respectively.

The electronic device 100 according to an embodiment of the present disclosure may determine the width w of the second shape 820, based on the shape of the target teeth. According to an embodiment, the electronic device 100 may determine the width w of the second shape 820, based on the x-axis length of the target teeth. For example, the electronic device 100 may determine the x-axis length of the target teeth as the width w of the second shape 820. For example, the electronic device 100 may determine a value obtained by adding a predetermined offset to the x-axis length of the target teeth as the width w of the second shape 820. According to another embodiment, the electronic device 100 may determine the width w of the second shape 820 as a predetermined value. For example, the electronic device 100 may determine the value (e.g., 10 mm) of the width w of the second shape 820 so that the second shape 820 has a size capable of being in contact with two teeth.

FIG. 9 illustrates a combined form of the first shape and the second shape according to an embodiment of the present disclosure.

The electronic device 100 according to an embodiment of the present disclosure may determine that a second shape 920 is combined with a first shape 910 so as to protrude from the first shape 910 by a predetermined length h1 (e.g., 1 mm) in the y-axis direction. According to an embodiment, the electronic device 100 may determine the length h1 by which the second shape 920 protrudes from the first shape 910 in the y-axis direction, based on the shape of the oral cavity. For example, the electronic device 100 may determine the length h1 by which the second shape 920 protrudes from the first shape 910 in the y-axis direction, based on the distance between antagonist teeth in the oral cavity. As the distance between the antagonist teeth in the oral cavity increases, the second shape 920 may protrude more from the first shape 910 in the y-axis direction.

FIG. 10 is a diagram illustrating a method for determining a depth of a second shape according to an embodiment of the present disclosure.

The electronic device 100 according to an embodiment of the present disclosure may determine a depth of a second shape 1020, based on an outer surface of a first shape 1010 and a portion of a surface 1030 of an antagonist tooth where the second shape 1020 is to be combined. The electronic device 100 may determine a first depth range d1 of a portion within a predetermined height m from the highest point of the outer surface of the first shape 1010. The electronic device 100 may determine a second depth range d2 of a portion of the surface 1030 of the antagonist tooth within a predetermined height n from the highest point, where the intraoral appliance 1000 will contact the second shape 1020 when mounted in the oral cavity. According to an embodiment, m and n may have the same value (e.g., 1 mm).

Referring to FIG. 10, the electronic device 100 may determine a depth d of the second shape 1020 based on the first depth range d1 and the second depth range d2. According to an embodiment, the electronic device 100 may determine a depth range obtained by combining the first depth range d1 and the second depth range d2 as the depth d of the second shape 1020. According to another embodiment, the electronic device 100 may determine, as the depth d of the second shape 1020, a depth range obtained by adding a predetermined offset to the depth range obtained by combining the first depth range d1 and the second depth range d2. The method for the electronic device 100 to determine the depth of the intraoral appliance 1000 is not limited to the method described above.

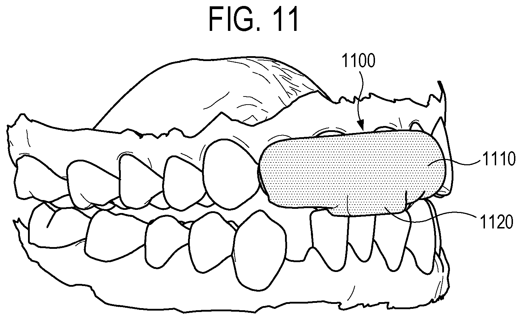

FIG. 11 illustrates a state in which an intraoral appliance generated based on third data is mounted within the oral cavity according to an embodiment of the present disclosure.

The electronic device 100 according to an embodiment of the present disclosure may generate first data on a first shape 1110 of an intraoral appliance 1100 and second data on a second shape 1120 according to the method described with reference to FIGS. 5 to 10. Based on the first and second data, the electronic device 100 may generate third data on a third shape of the intraoral appliance 1100 formed by combining the first shape 1110 and the second shape 1120. By mounting the intraoral appliance within the oral cavity, the user may prevent bruxism in various situations. For example, the intraoral appliance may prevent bruxism by generating a space between the user's maxilla and mandible to prevent direct contact.

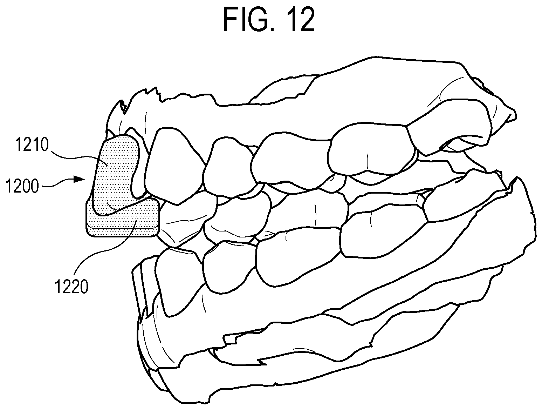

FIG. 12 illustrates a state in which an intraoral appliance generated based on the maxilla is mounted within the oral cavity according to an embodiment of the present disclosure.

According to an embodiment, a second shape 1220 of an intraoral appliance 1200, which is generated based on the maxilla, may be generated to extend lingually (palatally). When using the intraoral appliance 1200, contact between the second shape 1220 and the lips may open the lips in the wearing state, which may cause mouth breathing during sleep and thereby lead to dryness in the oral cavity. Dryness in the oral cavity due to mouth breathing may lead to various health problems, such as oral inflammation due to bacterial growth. Therefore, the position of the second shape 1220 may be adjusted to prevent this. For example, the second shape 1220 may be generated to extend lingually from the intraoral appliance 1200. When the second shape 1220 is generated to extend lingually rather than labially, contact between the second shape 1220 and the lips may be minimized, allowing the lips to close comfortably.

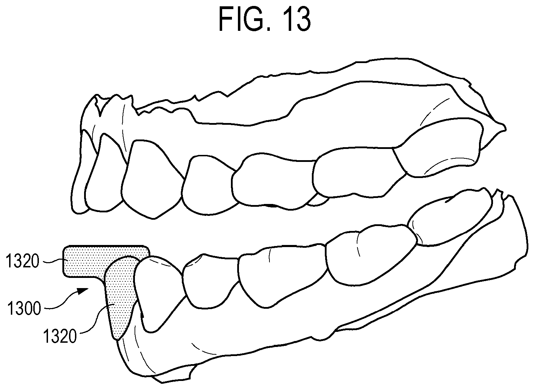

FIG. 13 illustrates a state in which an intraoral appliance generated based on the mandible is mounted within the oral cavity according to an embodiment of the present disclosure. The methods described in FIGS. 5 to 10 are based on the assumption that the first and second shapes of the intraoral appliance are generated based on the maxilla of the oral cavity. However, it will be readily apparent that the same description may be applied to an intraoral appliance 1300 generated based on the mandible of the oral cavity.

According to an embodiment, a second shape 1320 of an intraoral appliance 1300 generated based on the mandible may be generated to extend labially. If the second shape 1320 of the intraoral appliance 1300 generated based on the mandible protrudes lingually, it may interfere with tongue positioning, resulting in a foreign body sensation within the oral cavity. Therefore, by extending the second shape 1320 of the intraoral appliance 1300 labially, the foreign body sensation in the oral cavity may be minimized. In addition, for patients with a disrupted occlusion, the device size may be minimized to ensure stable mounting of the intraoral appliance in the oral cavity. Furthermore, if the width of the second shape 1320 is adjusted to increase the occlusal area with the antagonist tooth, occlusal pressure may be concentrated on one or two teeth, causing pain. However, by distributing the occlusal pressure across a plurality of teeth, the occlusal relationship between the intraoral appliance 1300 and the target teeth may be more stably maintained, thereby alleviating the patient's pain.

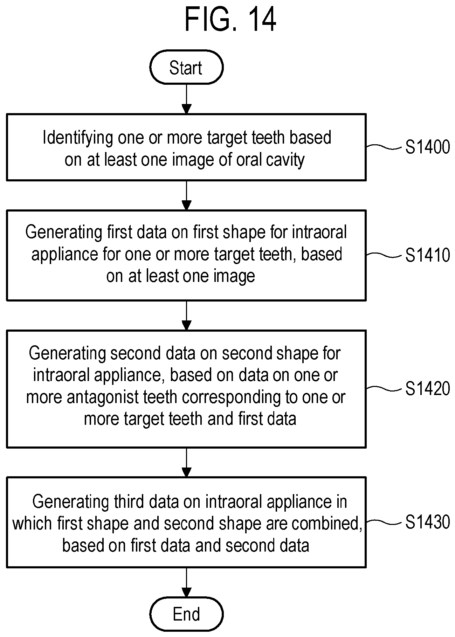

FIG. 14 is a flowchart of a method for generating an intraoral appliance according to an embodiment of the present disclosure.

In step S1400, the electronic device 100 according to an embodiment of the present disclosure may identify one or more target teeth based on at least one image of the oral cavity. The one or more target teeth may include first target teeth, which are inner teeth, and second target teeth, which are outer teeth. According to an embodiment, the target teeth may be four teeth in the anterior region.

The electronic device 100 according to an embodiment of the present disclosure may generate first data on a first shape for an intraoral appliance for one or more target teeth, based on at least one image, in step S1410.

The electronic device 100 according to an embodiment of the present disclosure may generate second data on a second shape for the intraoral appliance, based on data on one or more antagonist teeth corresponding to one or more target teeth and the first data, in step S1420.

In step S1430, the electronic device 100 according to an embodiment of the present disclosure may generate third data on the intraoral appliance in which the first shape and the second shape are combined, based on the first data and the second data.

Although the steps of the method or algorithm according to the present disclosure are described in a sequential order in the flowchart illustrated in FIG. 14, the steps may be performed in an order arbitrarily combined according to the present disclosure. The description of this flowchart does not exclude variations or modifications to the method or algorithm, nor does it imply that any step is essential or desirable. In an embodiment, at least some of the steps may be performed in parallel, iteratively, or heuristically. In an embodiment, at least some of the steps may be omitted, or other steps may be added thereto.

Various embodiments of the present disclosure may be implemented as software on a machine-readable storage medium. The software may be software for implementing various embodiments of the present disclosure. The software may be inferred from various embodiments of the present disclosure by programmers skilled in the art to which the present disclosure pertains. For example, software may be a program containing instructions (e.g., code or code segments) readable by a device. A device may be a device capable of operating according to instructions called from a storage medium, such as a computer. In an embodiment, the device may be an electronic device 100 according to embodiments of the present disclosure. In an embodiment, the processor of the device may execute the called instructions to cause components of the device to perform functions corresponding to the instructions. In an embodiment, the processor may be the processor 101 according to embodiments of the present disclosure. The storage medium may refer to any type of recording medium that stores data and may be read by the device. The storage medium may include, for example, a ROM, a RAM, a CD-ROM, a magnetic tape, floppy disks, optical data storage devices, etc. In an embodiment, the storage medium may be the memory 103. In an embodiment, the storage medium may also be implemented in a distributed form, such as in a network-connected computer system. Software may be distributed, stored, and executed on computer systems, etc. The storage medium may be a non-transitory storage medium. A non-transitory storage medium refers to a tangible medium, regardless of whether data is stored semi-permanently or temporarily, and does not include signals that are transmitted transitively.

Although the technical concept of the present disclosure has been described through various embodiments, the technical concept of the present disclosure encompasses various substitutions, modifications, and variations that may be made without departing from the scope of the present disclosure which may be understood by those skilled in the art to which the present disclosure pertains. In addition, it should be noted that such substitutions, modifications, and changes are intended to fall within the scope of the appended claims.

## Claims

1. A method performed by an electronic device, the method comprising:
identifying one or more target teeth based on at least one image of an oral cavity;
generating first data on a first shape for an intraoral appliance for the one or more target teeth, based on the at least one image;
generating second data on a second shape of the intraoral appliance, based on data on one or more antagonist teeth corresponding to the one or more target teeth and the first data; and
generating third data on the intraoral appliance in which the first shape and the second shape are combined, based on the first data and the second data.

2. The method of claim 1, wherein the generating the first data comprises:
generating data on an inner surface of the first shape, based on a shape of the one or more target teeth;
generating data on a contour of the first shape, based on one or more contour points and one or more connection points for the one or more target teeth; and
generating data on an outer surface of the first shape, based on the data on the inner surface and the data on the contour.

3. The method of claim 2, wherein the generating the first data comprises:
determining a first contour point for a first target tooth among the one or more target teeth in a first manner, based on the at least one image; and
determining a second contour point for a second target tooth among the one or more target teeth in a second manner different from the first manner.

4. The method of claim 3, wherein the one or more target teeth are four anterior teeth, the first target tooth is one of inner teeth among the four anterior teeth, and the second target tooth is one of outer teeth among the four anterior teeth.

5. The method of claim 3, wherein the determining the first contour point in the first manner comprises:
setting an x-coordinate value of the first contour point to an x-coordinate value of a center of a bounding box for the first target tooth; and
setting a y-coordinate value of the first contour point to a y-coordinate value of a reference line parallel to an occlusal surface of the oral cavity.

6. The method of claim 4, wherein the determining the second contour point in the second manner comprises
determining the second contour point, based on an intersection between a straight line connecting a center of the oral cavity and a center of a bounding box for the second target tooth and a surface of the outer teeth.

7. The method of claim 6, wherein the determining the second contour point in the second manner comprises:
determining a first point that is spaced apart from the center of the oral cavity in a y-axis direction by a value obtained by adding a predetermined value to a y-coordinate value of a reference line parallel to an occlusal surface of the oral cavity;
determining a second point that is spaced apart from a point existing within a bounding box for the second target tooth in the y-axis direction by a value obtained by adding a predetermined value to the y-coordinate value of the reference line parallel to the occlusal surface of the oral cavity;
determining a first intersection between a straight line connecting the first point and the second point and a surface of the second target tooth;
determining a third point that is spaced apart from the second point in an x-axis direction by a predetermined distance;
determining a second intersection between a straight line connecting the first point and the third point and the surface of the second target tooth; and
setting the second contour point to a farther one of the first intersection and the second intersection from the first point.

8. The method of claim 7, wherein the predetermined value represents movement of the second target tooth in a direction from the root toward the crown.

9. The method of claim 3, wherein the generating the first data comprises:
determining a reference point that is spaced a predetermined distance apart from a center of a bounding box for the second target tooth in a direction away from a center of the oral cavity; and
determining a point on a surface of the second target tooth, which is closest to the reference point, as a connection point.

10. The method of claim 1, wherein the generating the second data comprises:
determining a width indicating an x-axis length of the second shape;
determining a height indicating a y-axis length of the second shape; and,
determining a depth indicating a z-axis length of the second shape.

11. The method of claim 10, wherein the determining the width of the second shape comprises
determining the width of the second shape, based on an x-axis length of the one or more target teeth.

12. The method of claim 10, wherein the determining the height of the second shape comprises:
determining a length by which the second shape protrudes from the first shape in a direction toward the one or more antagonist teeth; and
determining the height of the second shape, based on the protruding length.

13. The method of claim 10, wherein the determining the depth of the second shape comprises:
determining a first depth range of a portion of an outer surface of the first shape where the second shape is to be combined, the portion being within a predetermined height from the highest point;
determining a second depth range of a portion of a surface of the one or more antagonist teeth that is to contact the second shape when the intraoral appliance is mounted in the oral cavity, the portion being within a predetermined height from the highest point; and
determining the depth of the second shape, based on the first depth range and the second depth range.

14. The method of claim 13, wherein the determining the depth of the second shape comprises:
determining the depth of the second shape by merging the first depth range and the second depth range and then adding a predetermined offset thereto.

15. The method of claim 1, wherein the generating the third data comprises:
in a case where the one or more target teeth are maxillary teeth, generating the third data so that a center of the second shape is positioned closer to a lingual side relative to the one or more target teeth when the intraoral appliance is mounted in the oral cavity.

16. The method of claim 1, wherein the generating the third data comprises:
in a case where the one or more target teeth are mandibular teeth, generating the third data so that a center of the second shape is positioned closer to a labial side relative to the one or more target teeth when the intraoral appliance is mounted in the oral cavity.

17. The method of claim 1, wherein the intraoral appliance is an NTI splint.

18. An electronic device comprising:
one or more processors; and
one or more memories configured to store instructions executed by the one or more processors,
wherein the instructions are configured to cause, when executed by the one or more processors, the one or more processors to execute a method according to any one of claims 1 to 17.

19. A non-transitory computer-readable recording medium recording instructions that, when executed by one or more processors, cause the one or more processors to perform an operation,
wherein the instructions are configured to cause the one or more processors to execute a method according to any one of Claims 1 to 17.
